Europäisches Patentamt
European Patent Office
Office européen des brevets

(11) Publication number: **0 207 635 A2**

# (12) EUROPEAN PATENT APPLICATION

(21) Application number: 86304161.2

(22) Date of filing: 02.06.86

(51) Int.Cl.⁴: C 07 D 493/10
B 41 M 5/12, B 41 M 5/26
//(C07D493/10, 311:00, 307:00)

(30) Priority: 28.06.85 GB 8516385

(43) Date of publication of application: 07.01.87 Bulletin 87/2

(84) Designated Contracting States: CH DE FR GB IT LI

(71) Applicant: IMPERIAL CHEMICAL INDUSTRIES PLC
Imperial Chemical House Millbank
London SW1P 3JF(GB)

(72) Inventor: Hughes, Nigel
7 Leonardin Close
Shaw OL2 7NH(GB)

(72) Inventor: Young Elliott
51 Birchfield Drive
Rochdale 011 4NY(GB)

(74) Representative: Stephenson, Kenneth et al,
Imperial Chemical Industries PLC Legal Department:
Patents PO Box 6 Bessemer Road
Welwyn Garden City Herts, AL7 1HD(GB)

## (54) Chromogenic compounds.

(57) A method for the manufacture of a chromogenic compound of general formula:

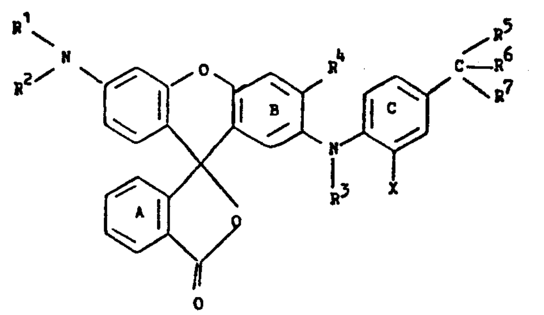

wherein $R^1$ and $R^2$, which may be the same or different, are alkyl, cycloalkyl, or aralkyl or $R^1$ and $R^2$, together with the nitrogen atom to which they are joined, form a heterocyclic ring;

$R^3$ is hydrogen, alkyl or optionally substituted aralkyl;

$R^4$ is hydrogen, alkyl, alkoxy or halogen;

$R^5$ is hydrogen or a hydrocarbyl radical and each of $R^6$ and $R^7$ is a hydrocarbyl radical, of which any aryl radicals represented by $R^5$, $R^6$ or $R^7$ may be substituted by halogen, nitro, cyano, alkyl; alkoxy or optionally substituted amino;

X represents hydrogen or a group of the formula $-CR^5R^6R^7$; and the benzenoid rings A, B and C may carry optional substituents, which comprises reacting, in the presence of concentrated inorganic acid, a compound of formula:

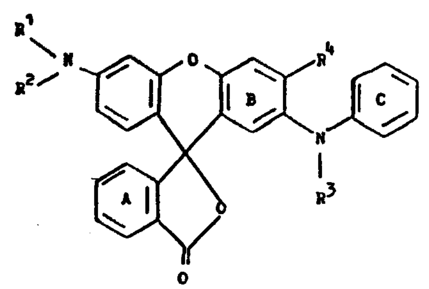

wherein $R^1$, $R^2$, $R^3$ and $R^4$ have the meanings given previously with a secondary or tertiary alcohol or an appropriately substituted olefin.

EP 0 207 635 A2

CHROMOGENIC COMPOUNDS

This invention relates to chromogenic compounds and in particular to a method for the manufacture of certain fluoran chromogenic compounds and to certain novel fluoran chromogenic compounds.

A chromogenic compound is a colourless or nearly colourless organic material which is capable of undergoing a colour-forming reaction on being brought into contact with a suitable co-reactant, for example an electron-accepting co-reactant such as an active clay material.

The colour-forming reaction may take place by contacting the chromogenic compound, preferably in the form of a solution in a suitable organic solvent, with the solid co-reactant, which may for example be coated onto or incorporated into a paper material. For many applications, a solution of the chromogenic compound in a suitable organic solvent is separated from the solid co-reactant by a membrane so that the colour develops only when the membrane is ruptured. For example, pressure-sensitive recording materials generally consist of the combination of (i) a base sheet having coated thereon microcapsules containing a solution in an organic solvent of the chromogenic compound and (ii) a receiving sheet coated with a suitable co-reactant. When pressure is applied to the base sheet, as for example by handwriting or typewriting, the membranes of the microcapsules at the point of pressure are ruptured, and a colour-forming reaction takes place as the solution of the chromogenic compound contacts the co-reactant. Variations of this general principle are possible, as for example a single recording sheet having a coating of micro-encapsulated chromogenic compound solution and a coating of co-reactant. Heat-sensitive recording materials function in a similar manner, the chromogenic compound and the co-reactant being separated by a medium which melts on the application of local heat as described for example in United States Patent No 3,539,375.

A number of chromogenic compounds based on the fluoran system are known. Thus, United Kingdom Patent Specification No.1332594 describes a process for producing fluoran compounds of the formula:

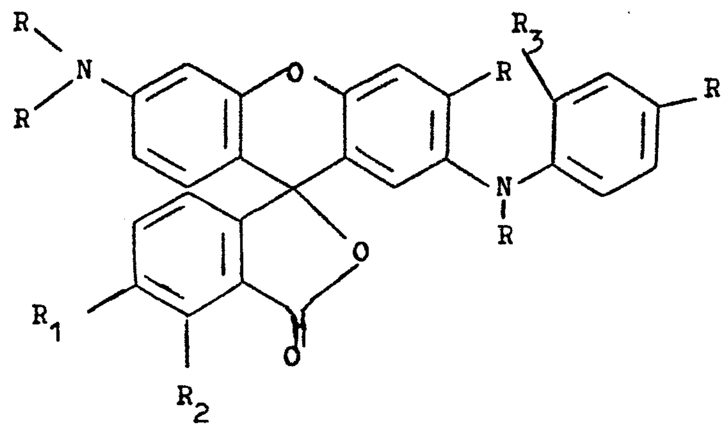

wherein one of $R_1$ and $R_2$ is hydrogen and the other is H, $CH_3$, $C_2H_5$, Cl, acetamido, $NH_2$ or $NO_2$; each R is H, $CH_3$ or $C_2H_5$ and $R_3$ is H or COOH by reacting an appropriate 2-hydroxy-4-amino-2'-carboxybenzophenone with an appropriate 4-anilinophenol in fuming sulphuric acid.

Using a similar method, Japanese Patent Publication 50082126 describes the preparation of a fluoran compound of the formula:

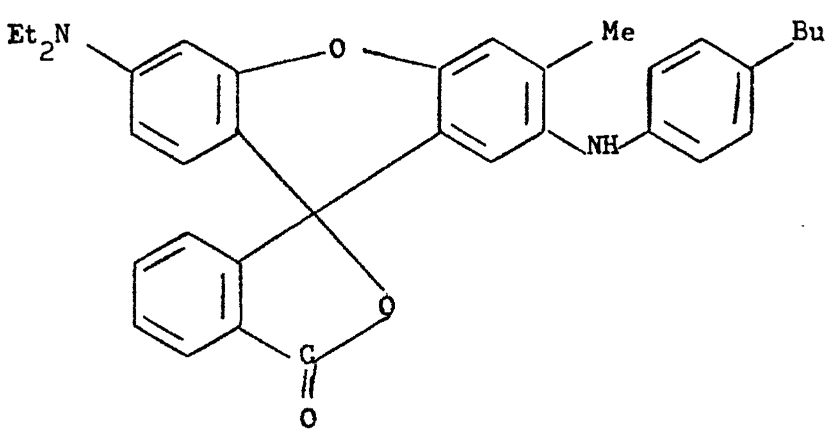

wherein Bu represents a tertiary butyl radical.

For certain fluoran compounds, the preparative method described above is not entirely satisfactory and in some cases may be quite unsuitable. It has now been found that these compounds may be prepared without difficulty using the method hereinafter described.

According to the present invention there is provided a method for the manufacture of a chromogenic compound of general formula:

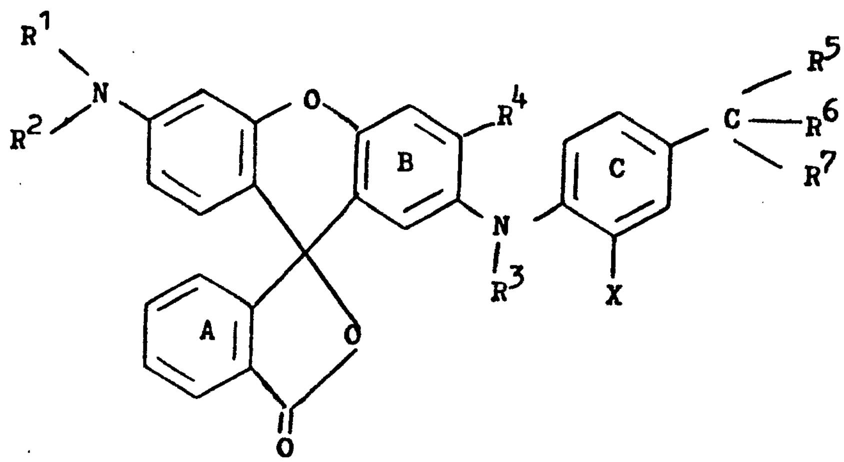

(I)

wherein $R^1$ and $R^2$, which may be the same or different, are alkyl, cycloalkyl or aralkyl or $R^1$ and $R^2$, together with the nitrogen atom to which they are joined, form a heterocyclic ring;

$R^3$ is hydrogen, alkyl or optionally substituted aralkyl;

$R^4$ is hydrogen, alkyl, alkoxy or halogen;

$R^5$ is hydrogen or a hydrocarbyl radical and each of $R^6$ and $R^7$ is a hydrocarbyl radical, of which any aryl radicals represented by $R^5$, $R^6$ or $R^7$ may be substituted by halogen, nitro, cyano, alkyl, alkoxy or optionally substituted amino;

X represents hydrogen or a group of the formula $-CR^5R^6R^7$;

and the benzenoid rings A, B and C may carry optional substituents, which comprises reacting, in the presence of concentrated inorganic acid, a compound of formula:

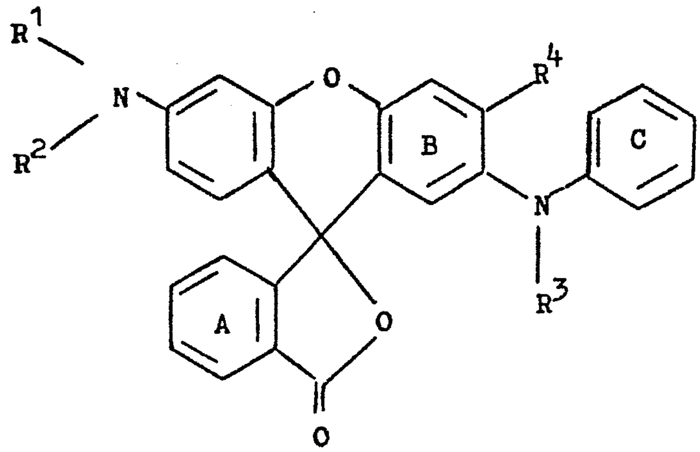

(II)

wherein $R^1$, $R^2$, $R^3$ and $R^4$ have the meanings given previously with a secondary or tertiary alcohol or an appropriately substituted olefin.

It has been found that, surprisingly, the above reaction gives the compound (I) in high yield and with a high degree of specificity of substitution in the desired 4- position in ring C, followed in some cases by substitution in the 2-position.

The nature of the optional substituents which may be present in the rings A, B and C is not critical to the present invention, and any such substituents conventional in the art or which would occur to the man skilled in art may be present in rings A, B and C. As examples of such substituents, there may be mentioned halogen, nitro, alkyl, alkoxy and hydroxy. In particular, ring A may be substituted by up to four halogen atoms

The secondary or tertiary alcohol may be represented by the formula:-

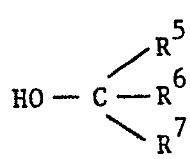

(III)

wherein $R^5$, $R^6$ and $R^7$, which may be the same or different, may be alkyl, cycloalkyl, optionally substituted aralkyl or optionally substituted

aryl or wherein one, but not more, of the groups $R^5$, $R^6$ and $R^7$ may be hydrogen. As alkyl groups, there may be mentioned especially lower alkyl groups containing from 1 to 6 carbon atoms, although there is no particular limit to the number of alkyl carbon atoms and an alkyl group containing as many as, for example, 12 carbon atoms may be utilised if desired. As optionally substituted aralkyl groups, there may be mentioned especially optionally substituted benzyl and as optionally substituted aryl groups, there may be mentioned especially the optionally substituted phenyl group. Optional substituents which may be present in the phenyl or benzyl ring include for example halogen, amino, alkylamino, dialkylamino, alkyl, alkoxy, cyano and nitro.

The olefin which is reacted with the compound of formula (II) is one carrying appropriate substituents for the introduction of a secondary or tertiary carbon atom at the 4-position of ring C. Suitable olefins may be represented by the formula:-

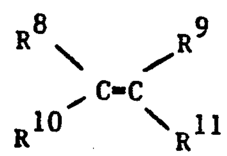

(IV)

It will be appreciated that the group $-CR^5R^6R^7$ is then generally represented by the formula

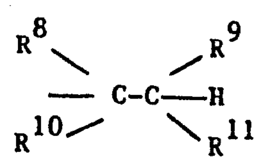

(V)

or

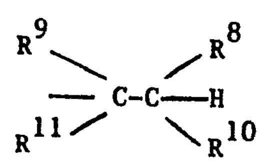

(VI)

or a mixture thereof depending on the mode of addition to the double bond.

In formula (IV) above, the substituents $R^8$, $R^9$, $R^{10}$ and $R^{11}$, which may the same or different, may be alkyl, cycloalkyl, optionally substituted aralkyl or optionally substituted aryl or up to three of $R^8$, $R^9$, $R^{10}$ and $R^{11}$ may be hydrogen. As alkyl groups there may be mentioned especially alkyl groups containing from 1 to 12 carbon atoms. As optionally substituted aralkyl groups, there may be mentioned especially optionally substituted benzyl and as optionally substituted aryl groups, there may be mentioned especially the optionally substituted phenyl group. Optional substituents which may be present in the phenyl or benzyl ring include for example halogen, amino, alkylamino, dialkylamino, alkyl, alkoxy, cyano and nitro.

It is not essential that a single compound of formula (IV) be used and there may be used for example a mixture of alkenes such as the isomeric mixture of $C_9$ alkenes commercially available as propylene trimer. The presence of an isomeric or other mixture in the group $-CR^5R^6R^7$, whether as a result of the use of a mixture of compounds of formula (IV) or whether as a result of the two possible modes of addition to the double bond illustrated as (V) and (VI) above, or as a result of rearrangement of the attacking species prior to reaction, is not disadvantageous. Indeed the presence of an isomeric mixture of groups at the 4-position of ring C will generally have the desirable effect of lowering the melting point of the chromogenic compound and increasing its solubility in those solvents commonly used for encapsulation.

The reaction between the compound of formula (II) and the compound of formula (III or IV) takes place readily in the presence of sulphuric acid or phosphoric acid which may optionally contain added phosphorus pentoxide. The concentration of the sulphuric acid preferably lies in the range 50% to 100% by weight and a concentration of about 80% by weight is especially preferred. The reaction temperature is preferably in the range from ambient temperature to about 100°C and a reaction temperature in the range 30°C to 70°C, for example about 50°C, is especially preferred. The reaction conveniently takes place under atmospheric pressure, although higher or lower pressures may be used if desired.

There is conveniently used essentially stoichiometric proportions of alcohol of formula (III) or olefin of formula (IV) to the compound of formula (II), although an excess of alcohol or olefin may be used if desired. There is preferably used an excess of acid. For example, when the acid is sulphuric acid, there is preferably used from 3 to 100 moles of sulphuric acid per mole of compound of formula (II).

The starting material of formula (II) may be prepared by known methods, for example an optionally substituted 3-aminophenol may be reacted with an optionally substituted phthalic anhydride in the presence of a suitable solvent such as boiling toluene to give the optionally substituted 2-(4-amino-2-hydroxybenzoyl)-benzoic acid of formula:-

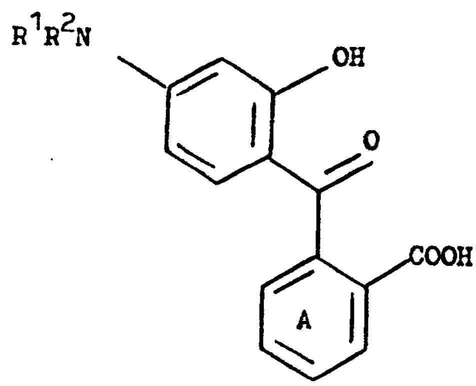

(VII)

The intermediate (VII) is then treated with optionally substituted 3-($R^4$)-4-(N-phenyl,N-$R^3$)-aminophenol in the presence of a dehydrating agent such as concentrated sulphuric acid to form the compound of formula (II). The compound of formula (II) may be isolated if desired, but if concentrated sulphuric acid has been used as dehydrating agent in its preparation, the compound of formula (II) is conveniently used in the process of the present invention without intermediate isolation.

Certain compounds of formula (I) are believed to be novel chromogenic compounds. In general such chromogenic compounds show improved properties as compared with compounds not having the $-CR^5R^6R^7$

group in the 4- position on ring C. Such improved properties may include an advantageous hypsochromic effect, an improved stability and light fastness, a reduced melting point and an increased solubility in those solvents commonly used for encapsulation.

Especially useful novel compounds are those of formula (I) in which $R^5$ is hydrogen or lower alkyl and each of $R^6$ and $R^7$ is an optionally substituted aryl radical, these compounds having particularly good fastness tolight.

The chromogenic compounds prepared in accordance with the present invention may be used in conventional manner. They may be used in conventional solvents known in the art such as alkylated biphenyls (for example monoisopropyl biphenyl), naphthalenes (for example partially hydrogenated naphthalene), terphenyls (which may be partially or totally hydrogenated), di esters of dicarboxylic acids (for example alkyl oxalates and phthalates) and polyhalogenated paraffins. The solution in a suitable organic solvent may be micro-encapsulated using conventional techniques, and the encapsulated system incorporated into pressure-sensitive or thermal copying materials.

Thus, the capsule walls can be formed evenly around the droplets of the colour former solution by coacervation; and the encapsulating material can consists of gelatin and gum arabic, as described e.g. in US patent 2800457. The capsules can also be formed from an aminoplast or a modified aminoplast by polycondensation, as described in British patent specifications 989264, 1156725, 1301052 and 1355124. Other suitable microcapsules are formed by interfacial polymerisation e.g. capsules formed from polyester, polycarbonate, polysulphonamide, polysulphonate, but in particular from polyamide or polyurethane.

The microcapsules containing the compounds of formula (1) can be used for the production of a wide variety of known kinds of pressure-sensitive copying material. The various systems differ substantially from one another in the arrangement of the capsules, of the colour reactions, and of the support. A preferred arrangement is that in which the encapsulated chromogenic compound is in the form of a

layer on the back of a transfer sheet and the developer is in the form of a layer on the face of a receiver sheet.

Another arrangement of the components is that wherein the microcapsules which contain the chromogenic compound, and the developer, are in or on the same sheet, in the form of one or more individual layers, or are present in the paper pulp.

Conventional co-reactants may be used for the chromogenic compounds prepared according to the present invention and as examples there may be mentioned active clays, (for example acid clay, attapulgite, zeolite or bentonite); a solid organic acid (such as succinic acid, tannic acid or benzoic acid); an acidic polymer (such as phenol-formaldehyde, phenol-acetylene polymer, residual acid group-containing styrene-maleic anhydride polymer or salicylic acid-formaldehyde polymer); and heavy metal salicylates (such as zinc salicylate). Bisphenol A is commonly used as a co-reactant in heat-sensitive applications.

The invention is illustrated by the following Examples in which all parts and percentages are by weight unless otherwise stated.

Example 1

This example illustrates the preparation of 3-diethylamino-6-methyl-7-(4-t-butyl-anilino)-fluoran of formula:-

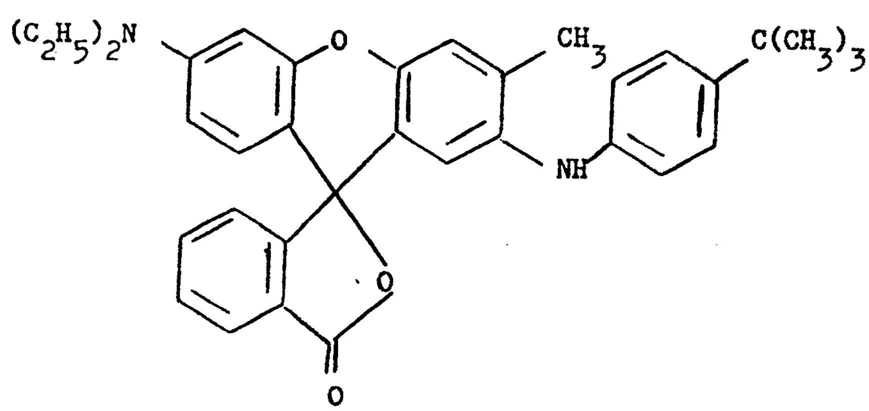

2.3 g of 3-diethylamino-6-methyl-7-anilinofluoran was added to

75 g of 80% w/w sulphuric acid at room temperature. After stirring for 15 minutes to effect solution, 1.5g of tertiary butanol was added and the temperature was raised to 50°C. After 2 hours the reaction was found to be complete and the solution was drowned into 200 g of ice and water and the mixture adjusted to pH 10 with sodium hydroxide. The precipitate was recovered by filtration and washed well with water before slurrying in methanol and filtering. The 2.2g of product was obtained after drying was recrystallised from toluene/petrol to give about 2g of white solid having a melting point of 186°C. The structure of the product was confirmed by nmr analysis which showed substitution in the 4- posiiton of ring C. Traces only of isomers having substitution in other than the 4- position in ring C were detected, but their total was too small to be measured.

The product of Example 1 was dissolved in toluene and the toluene solution was contacted with an acid clay surface to develop a reddish black shade.

The following Table gives further Examples of fluoran compounds prepared by the method of the invention. In the Table, the compounds are identified by substituents in the formula:

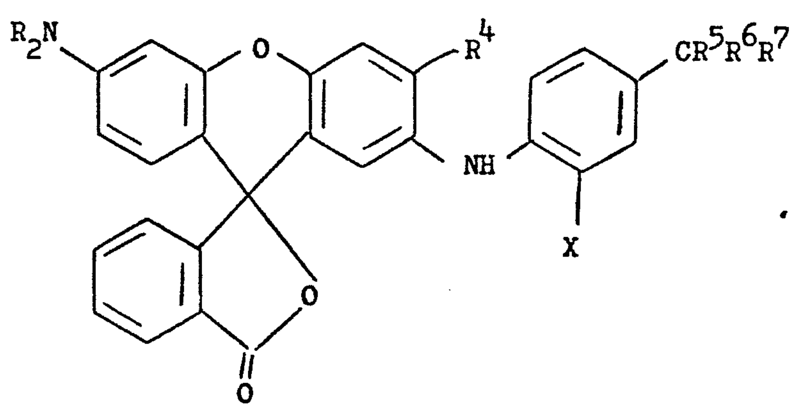

| Ex. | R | $R^4$ | $-CR^5R^6R^7$ | Shade | M.Pt. | X |
|---|---|---|---|---|---|---|
| 2 | Et | H | $-C(CH_3)_3$ | Greenish black | 183-5°C | H |
| 3 | Et | H | $-CH(Ph)_2$ | " | 133°C | $-CH(Ph)_2$ |
| 4 | Et | Me | $-CH(Ph)_2$ | Reddish black | 128°C | " |
| 5 | Et | H | $-C_9H_{18}$ (mixed isomers) | Greenish black | 100°C | H |
| 6 | Et | Me | $-C_9H_{18}$ (mixed isomers) | Reddish black | 120°C | H |
| 7 | Et | H | 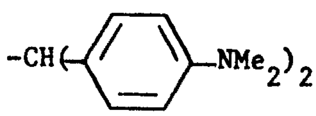 | Greenish black | 155°C | 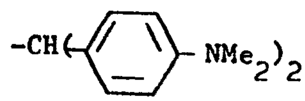 |
| 8 | Et | Me | " | Reddish black | 162°C | " |
| 9 | Et | Me | $-CH(CH_3)Ph$ | " | 169°C | |
| 10 | Et | Me | 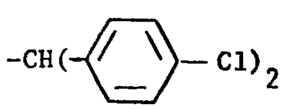 | " | 137°C | 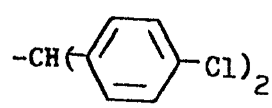 |
| 11 | Et | H | 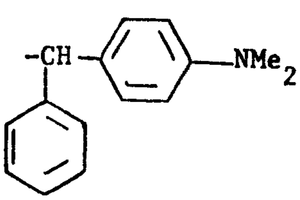 | Greenish black | 141°C | 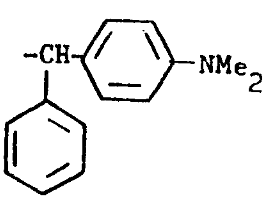 |

| Ex. | R | $R^4$ | $-CR^5R^6R^7$ | Shade | M.Pt. | X |
|---|---|---|---|---|---|---|
| 12 | Et | Me | 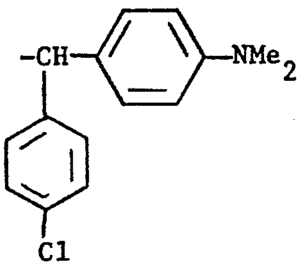 | Reddish black | 145°C | 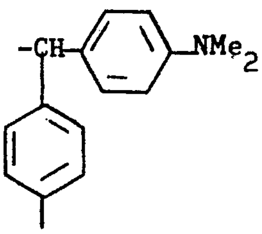 |
| 13 | Et | Me | 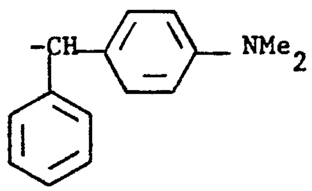 | " | 144°C | 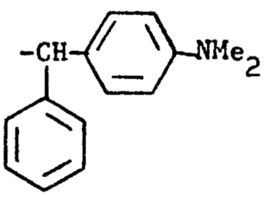 |
| 14 | Et | Me | 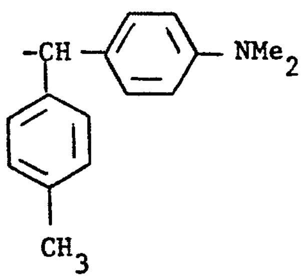 | " | 141°C | 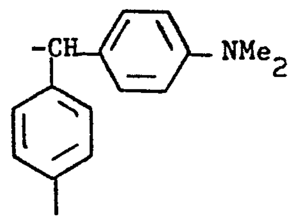 |
| 15 | Et | Me | 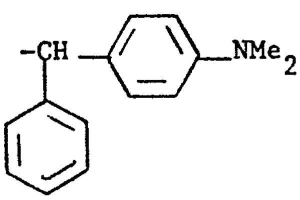 | " | 145°C | H |

In Examples 2-15, the $-CR^5R^6R^7$ group was introduced by using the appropriate secondary or tertiary alcohol except in Examples 5 and 6 where propylene trimer was used.

The following Table gives further Examples of fluoran compounds prepared by the method of the invention, the compounds being identified as previously. The Table also specifies the olefin used to introduce the $-CR^5R^6R^7$ substituent.

| Ex. | R | $R^4$ | $-CR^5R^6R^7$ | Olefin | Shade | M.Pt. | X |
|---|---|---|---|---|---|---|---|
| 16 | Et | Me | 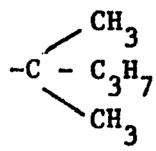 | 2-methyl-pent-1-ene | Reddish black | 148°C | H |
| 17 | Et | Me | 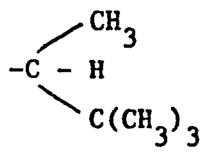 | 3,3-dimethyl-but-1-ene | " | 141°C | H |
| 18 | Et | Me | 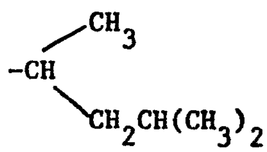 | 4-methyl-pent-1-ene | " | 145°C | H |
| 19 | Et | Me | 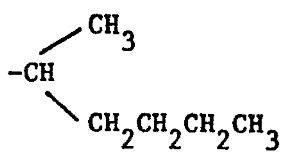 | 1-Hexene | " | 129°C | H |
| 20 | Et | Me | 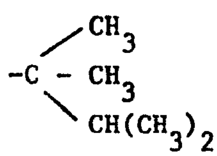 | 2,3-dimethyl-but-1-ene | " | 149°C | H |
| 21 | Et | Me | 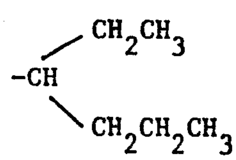 | 2-Hexene | " | 159°C | H |

In Examples 16-21, the specified $-CR^5R^6R^7$ substituent identifies the major isomeric component of the product.

CLAIMS

1. A method for the manufacture of a chromogenic compound of general formula:

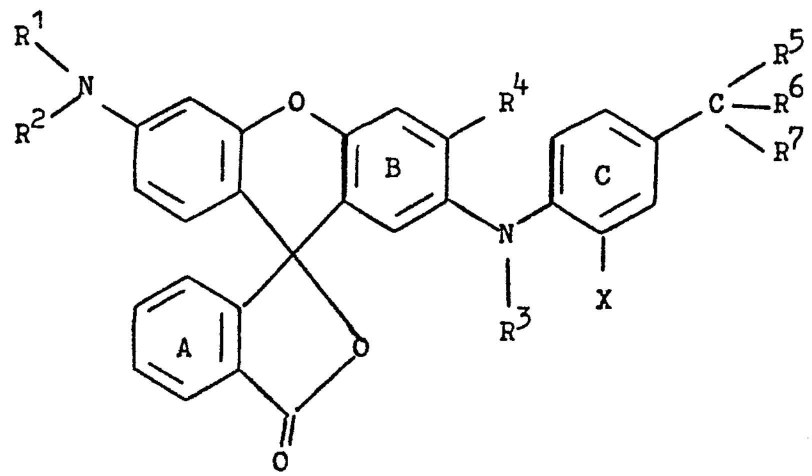

wherein $R^1$ and $R^2$, which may be the same or different, are alkyl, cycloalkyl or aralkyl or $R^1$ and $R^2$, together with the nitrogen atom to which they are joined, form a heterocyclic ring;

$R^3$ is hydrogen, alkyl or optionally substituted aralkyl;

$R^4$ is hydrogen, alkyl, alkoxy or halogen;

$R^5$ is hydrogen or a hydrocarbyl radical and

each of $R^6$ and $R^7$ is a hydrocarbyl radical, of which any aryl radicals represented by $R^5$, $R^6$ or $R^7$ may be substituted by halogen, nitro, cyano, alkyl, alkoxy or optionally substituted amino;

X represents hydrogen or a group of the formula $-CR^5R^6R^7$;

and the benzenoid rings A, B and C may carry optional substituents, which comprises reacting, in the presence of concentrated inorganic acid, a compound of formula:

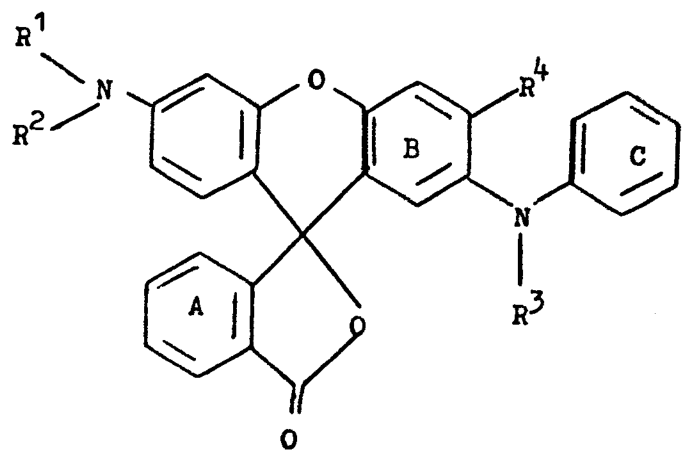

wherein $R^1$, $R^2$, $R^3$ and $R^4$ have the meanings given previously with a secondary or tertiary alcohol or an appropriately substituted olefin.

2. A method according to claim 1 wherein the secondary or tertiary alcohol has the formula:

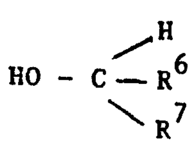

or

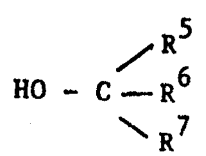

wherein each of $R^5$, $R^6$ and $R^7$, independently, represents an alkyl, cycloalkyl, optionally substituted aralkyl or optionally substituted aryl radical.

3. A method according to claim 1 wherein the olefin has the formula:

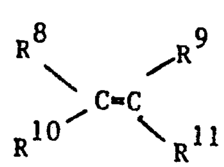

wherein each of $R^8$, $R^9$, $R^{10}$ and $R^{11}$, independently, represents an alkyl, cycloalkyl, optionally substituted aralkyl or optionally substituted aryl radical or up to three of $R^8$, $R^9$, $R^{10}$ and $R^{11}$ may be hydrogen.

4. A method according to claim 1 wherein the inorganic acid is sulphuric acid or phosphoric acid.

5. Pressure-sensitive or heat-sensitive recording material which contains a chromogenic compound prepared by the method of claim 1.

KS/BH

29.5.86.